# EUROPEAN PATENT SPECIFICATION

(11) **EP 3 038 557 B1**
(45) Date of publication and mention of the grant of the patent: **28.12.2016**
(21) Application number: 14753084.4
(22) Date of filing: 21.08.2014
(51) Int. Cl.: A61B 34/30, A61B 90/50, A61B 90/57

(54) **MEDICAL ROBOTIC AND/OR CATHETER CONTROL SYSTEM**
MEDIZINISCHES ROBOTER- UND/ODER KATHETERKONTROLLSYSTEM
SYSTÈME MÉDICAL POUR LE CONTRÔLE DES ROBOTS OU DES CATHÉTERS

(30) Priority: 30.08.2013 EP 13182492
(43) Date of publication of application: 06.07.2016
(73) Proprietor: Koninklijke Philips N.V., 5656 AE Eindhoven (NL)
(72) Inventor: JANSSEN, Erik Johannes Maria, 5656 AE Eindhoven (NL); VAN DIJK, Erik Martinus Hubertus Petrus, 5656 AE Eindhoven (NL)
(74) Representative: Gravendeel, Cornelis
(86) International application number: PCT/EP2014/067786
(87) International publication number: WO 2015/028375

(56) References cited:
- EP-A1- 2 218 396
- WO-A2-2004/082553
- DE-A1-102005 053 030
- DE-U1- 8 905 588
- US-A- 4 901 967
- US-A1- 2010 074 681
- US-A1- 2010 305 502

## Description

### FIELD OF THE INVENTION

The invention relates to a medical system and medical method for applying a medical procedure to a living being.

### BACKGROUND OF THE INVENTION

US 4,901,967 discloses a hospital service column for association with an equipment transport bracket including a bracket post. The hospital service column comprises a pivotable support arm having a latching block assembly disposed for locking reception of the transport bracket, wherein the block assembly includes a post receiving tube adapted to receive the bracket post for pivotable reception of the bracket and the block assembly has a latch plate and a locking pin assembly. The hospital service column further comprises a piston and cylinder assembly in association with the support arm for selective powered positioning of the support arm, wherein the support arm is mounted to a shaft coupled to the piston and cylinder assembly. Moreover, the hospital service column comprises a selectively operable valve means for operation of the piston and cylinder assembly and a housing sized to contain the piston and cylinder assembly and including service outlet means for the communication of power and gas sources.

US 2010/0305502 A1 discloses a device motion control mechanism for moving at least one elongate medical device. The mechanism comprises a drive base having a plurality of slots, wherein each slot has an open top and opposed sides therein, a first plurality of pairs of opposed wheels on opposite sides of a corresponding slot, and a drive mechanism configured for connection to a plurality of motors, each motor configured to turn at least one wheel of a corresponding pair of opposed wheels. The mechanism further comprises a rotary sleeve into which the device may pass for engagement with the sleeve, wherein the sleeve together with the device may be axially rotated by means of engagement with a second plurality of opposed wheels. The mechanism first comprises, for each first pair of wheels, a cover movably mounted on the drive base for movement between a loading position in which the top of a slot is open to allow a portion of at least one elongate device to be inserted into the open slot between the corresponding pairs of wheels and a drive position in which the cover at least partially blocks the top of a slot to retain at least one elongate device therein.

US 2010/0074681 A1 discloses a latching assembly for automatically locking a transport bracket to a service column, wherein the latching assembly comprises a service column latching assembly and transport bracket latching assembly. The service column latching assembly comprises a male coupling depending from the service column, a coupling insert depending from the male coupling, wherein the coupling insert defines an aperture extending from a coupling insert interior to a coupling insert exterior, and a retaining element movably retained within the aperture. The transport bracket latching assembly comprises a tubular cage having an interior groove and a latch positioned inside the cage so that, upon insertion of the latch into the coupling insert interior, the latch forces the retaining element into the interior groove to maintain attachment of the transport bracket latching assembly to the service column latching assembly.

The two-part form of appended claim 1 is based on US 2010/0074681 A1.

DE 89 05 588 U1 and DE 10 2005 053 030 A1 disclose x-ray C-arm systems attached to a floor or a ceiling of a room. EP 2 218 396 A1 discloses a robotic manipulator for remotely maneuvering catheters, wherein the robotic manipulator is attached to a patient table, and WO 2004/082553 A2 discloses a radial arm system for patient care equipment like infusion equipment, monitors, et cetera.

US 8,257,303 B2 discloses a robotic catheter system comprising an operator control station located remotely from an operating table to which an instrument driver and an instrument are attached by an instrument driver mounting brace. A communication link transfers signals between the operator control station and the instrument driver.

Since the operating table carries the instrument driver and the instrument, the patient weight capacity of the operating table is reduced.

### SUMMARY OF THE INVENTION

It is an object of the present invention to provide a medical system for applying a medical procedure to a living being, wherein the weight capacity of a support means carrying the living being can be increased.

In a first aspect of the present invention a medical system for applying a medical procedure to a living being is presented, wherein the medical system comprises:
- a medical device for performing the medical procedure, wherein the medical device is adapted to perform a movement relative to a reference device for applying the medical procedure, wherein the medical device includes a medical robotic device and/or a catheter control device,
- an attaching system for attaching the medical device to a wall and/or a ceiling of a room, wherein the attaching system is a suspension system for suspending the medical device on the wall and/or the ceiling of the room,
- a fixation element for rigidly attaching the medical device to the reference device, and
- a passive compliant element between the attaching system and the medical device, wherein the compliant element is adapted to provide compliance when the medical device is moved relative to the attaching system..

Since the medical device for performing the medical procedure is attached to the wall and/or the ceiling of the room, the weight of the medical device does not need to be completely carried by a support means for supporting the living being. Moreover, since the medical device can be rigidly attached to the reference device by using the fixation element, i.e. since a stiff connection between the medical device and the reference device can be provided, a high accuracy of moving at least a part of the medical device relative to the reference device and a high positioning stability can still be guaranteed, thereby allowing for an accurate application of the medical procedure. An increased weight capacity of the support means can therefore be provided, while the medical procedure can still very accurately be performed. In particular, if the reference device and the support means are both a patient table, on which a patient is lying, the medical procedure can still accurately be applied to the patient by using the medical device, although the medical device is not completely carried by the patient table, but mainly by the wall and/or ceiling of the room, thereby allowing for an increased weight capacity of the patient table.

The living being is a person or an animal. The medical device includes a medical robotic device like a surgical robot, which may also be regarded as being an operation robot, and/or a catheter control device. The attaching system, the fixation element and the compliant element are preferentially adapted such that the reference device carries some kilograms, but not more than 10 kg, whereas the remaining weight is carried by the wall and/or ceiling via the compliant element and the attaching system. The overall weight to be carried by the reference device and the wall and/or ceiling via the compliant element and the attaching system maybe in the range of 100 kg. The medical system further comprises a compliant element between the attaching system and the medical device, which is preferentially a spring element. The spring element may be a mechanical or pneumatic spring. The compliant element, which may also be regarded as being a gravity compensator, allows for small movements of the medical device with respect to the attaching system, without necessarily modifying the configuration of the attaching system, before applying the medical procedure, for instance, while rigidly attaching the medical device to the reference device, and/or during the medical procedure. In particular, the compliant element is preferentially adapted to allow for movements of the medical device relative to the attaching system of at least 10 cm and further preferred of 30 cm or more. The compliant element is preferentially a compliant element, particularly a passive compliant element, which provides compliance, if a user moves the medical device relative to the attaching system, for instance, by directly moving the medical device, especially while positioning the medical device relative to the reference device, or by moving the reference device, if the medical device has already been rigidly attached to the reference device.

The attaching system is a suspension system for suspending the medical device on a wall and/or a ceiling of a room. The attaching system is preferentially adapted to movably attach the medical device such that the medical device is movable to different locations and/or into different positions. This allows the medical device to be moved to a desired location and/or into a desired position, wherein at this location and optionally also in this position the medical device can be rigidly attached to the reference device by using the fixation element.

Preferentially, for a first session the medical device is rigidly attachable to the reference device by the fixation element for providing a stiff connection between the medical device and the reference device, wherein, after the medical device has been detached from the reference device, for a second session the medical device is again rigidly attachable to the reference device by the fixation element for providing a stiff connection between the medical device and the reference device, wherein the fixation element is adapted such that the spatial relation between the medical device and the reference device is the same in the first and second sessions. For instance, the fixation element can comprise rigid elements such that the same configuration of the fixation element can be used in the first and second sessions, in order to not change the spatial relation between the medical device and the reference device in the first and second sessions. Thus, the spatial relation between the medical device and the reference device is reproducible such that the second session can directly start, after the medical device has been attached to the reference device, without, for instance, determining the spatial relation between the medical device and the reference device at the beginning of the second session.

The fixation element is preferentially adapted such that the medical robotic device and/or the catheter control device can be positioned with respect to the reference device with an accuracy being equal to or smaller than 1 mm and/or 0.1 degrees at the position where the medical robotic device and/or the catheter control device is attached to the reference device. It is further preferred that the medical device and the fixation element are adapted such that a distal tip of the medical robotic device and/or of a catheter controlled by the catheter control device can be positioned with respect to the reference device with an accuracy being equal to or smaller than 1 mm and/or 0.1 degrees.

The fixation element may be adapted to allow for a fixation of the medical device in two steps, wherein in a first step the medical device may be arranged relative the reference device and in a second step the medical device may be fixed in the position in which the medical device has been arranged relative to the reference device. For instance, the fixation element can comprise a) two dowel pins fixed to one of the reference device and the medical device and b) two holes fixed to the other of the reference device and the medical device, wherein one of the holes may be a slotted hole. The two dowel pins can penetrate the two holes for accurately positioning the medical device relative to the reference device in the first step. In the second step the medical device can be fixated relative to the reference device by using known fixation means like spring loaded bayonet quick locks, quick locks, clamps, twist locks, pneumatic means, et cetera. In a further example the fixation element can comprise a) two bearing balls fixed to one of the reference device and the medical device and b) two slots, wherein a first slot may be a tapered slot which holds a bearing ball at the location of the first slot and which may have a circular horizontal cross section and wherein a second slot may be an elongated grooved slot limiting a movement in one direction. In the first step the medical device may be arranged relative to the reference device by positioning the bearing balls on the two slots. In the second step this arrangement can be fixed by using known fixation means like spring loaded bayonet quick locks, clamps, twist locks, pneumatic means, et cetera.

The medical system preferentially further comprises an imaging device for generating an image of the living being during the medical procedure. The imaging device is preferentially an x-ray C-arm system for generating x-ray projection images of the living being and optionally also of a part of the medical device like a needle or a catheter within the living being.

In another aspect a medical method for applying a medical procedure to a living being by using the medical system as defined in claim 1 is presented, wherein the medical method comprises:
- attaching the medical device to a wall and/or a ceiling of a room by using the attaching system, wherein the attaching system is a suspension system for suspending the medical device on the wall and/or the ceiling of the room, wherein the medical device is adapted to perform a movement relative to a reference device for applying the medical procedure, wherein the medical device includes a medical robotic device and/or a catheter control device,
- rigidly attaching the medical device to the reference device by using the fixation element, thereby moving the medical device relative to the attaching by using the compliant element, and
- performing the medical procedure by using the medical device.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a medical system for applying a medical procedure to a living being, and
Fig. 2 shows a flowchart exemplarily illustrating an aspect of a medical method for applying a medical procedure to the living being.

### DETAILED DESCRIPTION OF EMBODIMENTS

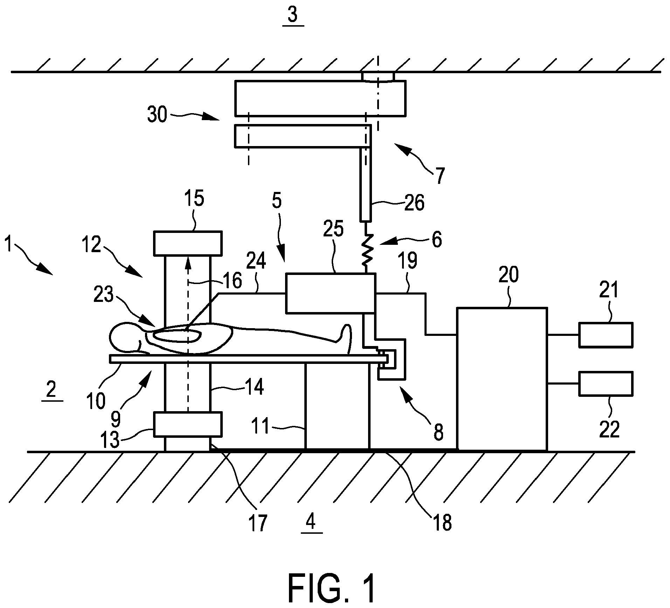

Fig. 1 shows schematically and exemplarily an embodiment of a medical system for applying a medical procedure to a living being. In this embodiment the medical system 1 is adapted to apply a surgical operation to a person 23 lying on a patient table 9 comprising a table plate 10 and a table leg 11. Preferentially, the height of the table leg 11 is modifiable and the table plate 10 is translatable with respect to the table leg 11, in order to position the patient 23 as desired.

The surgical procedure is performed by a surgical robot 5 comprising a surgical instrument 24 like a needle or a catheter introduced into the body of the patient 23 and a surgical instrument driving unit 25 for driving the surgical instrument 24. The patient table can be regarded as being a reference device, relative to which the surgical instrument 24 and, thus, the surgical robot 5, i.e. in this embodiment a part of the surgical robot 5, is moved during the medical procedure. The surgical robot 5 is controlled by a controlling device 20, which is located remotely from the surgical robot 5 and which is connected with the surgical robot 5 via an electrical connection 19. The controlling device 20 is adapted to control the surgical robot 5 in accordance with control data input into the controlling device 20 via an input unit 21. The input unit 21 comprises, for instance, a keyboard, a joystick, a touch screen, a computer mouse, et cetera, which allow an operator to control the surgical robot 5 via the controlling device 20 as desired. In this embodiment the electrical connection 19 is a wired electrical connection. However, in another embodiment the electrical connection may also be a wireless connection or a mechanical connection.

The medical system 1 further comprises an attaching system 7 for attaching the surgical robot 5 to a ceiling 3 of a room 2. The attaching system 7 is a suspension system for suspending the surgical robot 5 on the ceiling 3, wherein the suspension system moveably attaches the surgical robot 5 to the ceiling 3 such that the surgical robot 5 is moveable to different locations and/or into different positions. For instance, the suspension system 7 can comprise several longish segments 30, wherein adjacent ends of the longish segments 30 are connected to each other via hinges such that the longish segments 30 are rotatable relative to each other. One end of one of the longish segments 30 can also be rotatably connected to the ceiling 3. Moreover, at least longish segment can be configured such that its length is modifiable. For example, at least one segment can be made of several tubes having different diameters and being inserted into each other for allowing the operator to modify the length of the respective segment.

In this embodiment the attaching system 7 further comprises a vertically oriented rod 26 connecting an end of a segment 30 with a compliant element 6 being, in this embodiment, a spring element. The spring element 6 may be, for instance, a mechanical spring or a pneumatic spring.

The medical system 1 further comprises a fixation element 8 for rigidly attaching the surgical robot 5 to the patient table 9, in order to provide a stiff connection between the surgical robot 5 and the patient table 9. The fixation element 8 may be a metallic element, which can be attached to the patient table 9 by using screws or other elements providing a rigid attachment to the patient table 9. In an embodiment, for a first session the surgical robot 5 is rigidly attached to the patient table 9 by the fixation element 8 for providing a stiff connection between the surgical robot 5 and the patient table 9, wherein, after the surgical robot 5 has been detached from the patient table 9, for a second session the surgical robot 5 is again rigidly attached to the patient table 9 by the fixation element 8 for providing a stiff connection between the surgical robot 5 and the patient table 9, wherein the fixation element 8 is adapted such that the spatial relation between the surgical robot 5 and the patient table 9 is the same in the first and second sessions. For instance, the fixation element 8 can comprise rigid elements such that the same configuration of the fixation element 8 can be used in the first and second sessions, in order to not change the spatial relation between the surgical robot 5 and the patient table 9 in the first and second sessions. Thus, the spatial relation between the surgical robot 5 and the patient table 9 is reproducible such that the second session can directly start, after the surgical robot 5 has been attached to the patient table 9, without, for instance, determining the spatial relation between the surgical robot 5 and the patient table 9 at the beginning of the second session.

The medical system 1 further comprises an imaging device 12 for generating an image of the surgical instrument 24 within the patient 23. The image can be provided to the controlling device 20 via a data connection 18, which is preferentially a wired data connection, wherein the generated image can be shown on a display 22. The operator can see the surgical instrument 24 within the patient 23 relative to the inner anatomy of the patient 23 on the display 22 and the operator can then control the surgical robot 5 via the controlling device 20 by using the input unit 21 based on the position and orientation of the surgical instrument 24 within the anatomy of the patient 23 as shown by the image on the display 22. In this embodiment the imaging device 12 is an x-ray C-arm device comprising an x-ray source 13 emitting x-rays 16 traversing the patient 23 and an x-ray detector 15 detecting the x-rays 16 after having traversed the patient 23. The x-ray source 13 and the x-ray detector 15 are mounted on a C-arm 14. The C-arm 14 is movable with respect to a carrying structure 17 for carrying the C-arm 14. The carrying structure 17 is arranged on the floor 4 of the room 2 and may be fixed to or movable relative to the floor 4. In another embodiment the C-arm may be attached to the ceiling via a corresponding carrying element attached to the ceiling. The imaging device 12 generates x-ray projection images of the patient 23, which are provided to the controlling device 20 and shown on the display 22.

All components of the medical system 1 and the patient 23 may be registered with respect to the patient table 9. For instance, the imaging device 12 can be registered with respect to the patient table 9 based on features of the patient table 9, which are arranged at known locations within a reference coordinate system defined by the patient table 9 and which are visible in the projection images generated by the imaging device 12. The features of the patient table 9 can be metallic markers or other markers being visible in x-ray projection images. In other embodiments the imaging device 12 and the patient table 9 may also be registered with respect to each other in another way. For instance, instead of using features of the patient table, during the registration procedure a phantom can be attached to the patient table at a known location within a reference coordinate system defined by the patient table 9, wherein the phantom can comprises features visible in the projection images generated by the imaging device 12. The registration can then be performed based on the known location of the phantom relative to the patient table 9, i.e. in the reference coordinate system, and based on the positions of the features of the phantom in the projection images. The surgical robot 5 may be registered with the patient table 9 by registering the surgical robot 5 with the imaging device 12, wherein an image generated by the imaging device 12 and showing at least a part of the surgical robot 5 like a part of the surgical instrument 24 may be used, and by using the determined registration between the imaging device 12 and the patient table 9. Also the registration between the surgical robot 5 and the patient table 9 may be performed in another way. For instance, the surgical robot 5 and the patient table 9 may be registered based on a known geometrical configuration of the fixation element 8 providing the stiff connection.

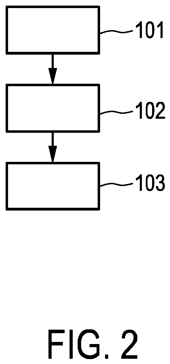

In the following an aspect of a medical method for applying a medical procedure to a living being by using the medical system 1 will exemplarily be described with reference to a flowchart shown in Fig. 2.

In step 101 the surgical robot 5 is attached to the ceiling 3 of the room 2 by using the attaching system 7 with the compliant element 6. In step 102 the surgical robot 5 is rigidly attached to the patient table 9 by using the fixation element 8, whereafter the surgical procedure is performed by using the surgical robot 5 in step 103. In particular, in step 103 projection images are generated by the imaging device 12 and shown on the display 22, while an operator controls the surgical instrument 24 of the surgical robot 5 via the controlling device 20 by using the input unit 21.

If the surgical robot would completely be carried by the patient table, the patient weight capacity of the patient table would be reduced. Moreover, in case of removal or placement of the surgical robot a person like a nurse would need to carry the total weight of the surgical robot, which may be about 20 kg or the like, which would not be optimal from an ergonomic and workflow point-of-view. Furthermore, storage space would be needed in the room for the surgical robot, when it is not used, for instance, on a cart or another storage location, thereby requiring valuable space in the operating room. In addition, completely carrying the surgical robot by the patient table would complicate the workflow in the operating room, because generally a table top of the patient table is detachable and in a normal workflow between two different medical procedures the table top will be removed to transport the patient. This would mean that the heavy surgical robot would need to be removed manually in between each medical procedure.

To overcome at least some of these drawbacks of carrying the entire weight of the surgical robot by the patient table, the medical system described above with reference to Fig. 1 comprises a surgical robot, which is suspended on the ceiling via a pendant system, i.e. via the suspension system. When the surgical robot is not being used, it can be moved away from the patient table. When it is being used, the surgical robot can be moved to a location above the patient table and there it can be docked with a stiff connection on the patient table by using the fixation element, while the surgical robot is still compliantly connected to the pendant with a gravity compensator, i.e. with the compliant element. This implies that the patient table is not carrying the full weight of the surgical robot, but just the part of the weight which is not compensated for by the gravity compensator on the pendant. This medical system offers a very convenient and economic solution for putting the surgical robot in its working position, wherein the patient table and the operator, who positions the surgical robot in its working position, do not need to carry the full weight of the surgical robot. On the one hand that reduces the required load capacity of the patient table and on the other hand that opens the door to heavier surgical robots, which may be able to perform more sophisticated procedures. Since the surgical robot still docks to the patient table via a stiff connection, the positioning accuracy and its position stability with respect to the patient is still guaranteed.

In the embodiment described above with reference to Fig. 1, the medical system comprises a ceiling or wall suspension system, which can also be regarded as being a pendant system, a compliant connection with gravity compensation between the suspension system and the equipment to be positioned, which is the surgical robot in this embodiment, the equipment and a stiff connection between the equipment and a reference system, i.e. the reference device, which is the component which determines the position and orientation of the equipment. In the embodiment described above with reference to Fig. 1 the reference system is the patient table. The majority of the weight of the equipment is carried by the suspension system via the compliant connection. The suspension system allows for a movement of the equipment over the field of use, when the equipment is in operation as well as during standby or parking. When the equipment is in use and a high position accuracy is required between the patient table and the equipment, the equipment will be docked to the patient table, i.e. to the reference system, via the stiff docking mechanism, i.e. via the fixation element. Still the majority of the weight of the equipment is carried by the suspension system. This means that the patient table only needs to carry a fraction of the total weight of the equipment. Since the equipment is compliantly connected to the ceiling system, the position of the equipment will be determined by the position of the patient table and not by the position of the suspension system, when the equipment is rigidly attached to the patient table by using the fixation element. Moreover, table movements are still possible, since the suspension system and the compliant element allow for a movement of the equipment rigidly attached to the patient table.

Although in the embodiment described above with reference to Fig. 1 the equipment is a surgical robot, in other embodiments the equipment can also be another heavy component, which requires a high positioning accuracy with respect to the patient and which is due to its size and/or weight difficult to handle manually. This equipment is preferentially equipment being adapted to be used in the surgery domain or in the cardiovascular domain, in particular, in the minimal invasive surgery domain. The equipment can include, for example, an operation robot like the above described surgical robot, a robotic arm, a catheter control system, a navigation system, et cetera, which may be remotely controllable by an operator by a controlling device like the controlling device described above with reference to Fig. 1.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope. The invention relates to a medical system for applying a medical procedure. The medical system comprises a medical device like a surgical robot with a surgical instrument for performing the medical procedure, wherein the medical device is adapted to perform a movement, for instance, a movement of the surgical instrument, relative to a reference device like a patient table for applying the medical procedure. The medical device is attached to a wall and/or a ceiling of a room by an attaching system and rigidly attached to the reference device by a fixation element. Due to the attachment to the wall and/or the ceiling, the weight of the medical device does not need to be completely carried by, for instance, the patient table, wherein, due to the rigid attachment to the reference device, the medical procedure can still very accurately be performed.

## Claims

1. A medical system for applying a medical procedure to a living being, wherein the medical system (1) comprises:
- a medical device (5) for performing the medical procedure, wherein the medical device is adapted to perform a movement relative to a reference device (9) for applying the medical procedure,
- an attaching system (7) for attaching the medical device (5) to a wall and/or a ceiling (3) of a room, wherein the attaching system (7) is a suspension system for suspending the medical device (5) on the wall and/or the ceiling (3) of the room (2),
- a fixation element (8) for rigidly attaching the medical device (5) to the reference device (9), and
- a passive compliant element (6) between the attaching system (7) and the medical device (5), wherein the compliant element (6) is adapted to provide compliance when the medical device (5) is moved relative to the attaching system (7).
**characterised in that** the medical device (5) includes a medical robotic device and/or a catheter control device.

2. The medical system as defined in claim 1, wherein the compliant element (6) comprises a spring element.

3. The medical system as defined in claim 1, wherein the compliant element (6) is adapted to allow for movements of the medical device relative to the attaching system of at least 10 cm.

4. The medical system as defined in claim 1, wherein the attaching system (7) is adapted to movably attach the medical device (5) such that the medical device (5) is movable to different locations and/or into different positions.

5. The medical system as defined in claim 1, wherein the reference device (9) is a support means for supporting the living being (23), wherein the fixation element (8) is adapted to be rigidly attached to the support means.

6. The medical system as defined in claim 5, wherein the support means is a patient table.

7. The medical system as defined in claim 1, wherein for a first session the medical device (5) is rigidly attachable to the reference device (9) by the fixation element (8) for providing a stiff connection between the medical device (5) and the reference device (9), wherein, after the medical device has been detached from the reference device, for a second session the medical device (5) is again rigidly attachable to the reference device (9) by the fixation element (8) for providing a stiff connection between the medical device (5) and the reference device (9), wherein the fixation element (8) is adapted such that the spatial relation between the medical device (5) and the reference device (9) is the same in the first and second sessions.

8. The medical system as defined in claim 1, wherein the medical system (1) further comprises an imaging device (12) for generating an image of the living being (23) during the medical procedure.

## Patentansprüche

1. Medizinisches System zum Anwenden einer medizinischen Prozedur auf ein Lebewesen, wobei das medizinische System (1) Folgendes umfasst:
- eine medizinische Vorrichtung (5) zum Durchführen der medizinischen Prozedur, wobei die medizinische Vorrichtung dafür eingerichtet ist, eine Bewegung in Bezug zu einer Referenzvorrichtung (9) auszuführen, um die medizinische Prozedur anzuwenden,
- ein Anbringungssystem (7) zum Anbringen der medizinischen Vorrichtung (5) an einer Wand und/oder einer Decke (3) eines Raums, wobei das Anbringungssystem (7) ein Aufhängungssystem zum Aufhängen der medizinischen Vorrichtung (5) an der Wand und/oder der Decke (3) des Raums (2) ist,
- ein Befestigungselement (8) zum starren Befestigen der medizinischen Vorrichtung (5) an der Referenzvorrichtung (9), und
- ein passives nachgiebiges Element (6) zwischen dem Anbringungssystem (7) und der medizinischen Vorrichtung (5), wobei das nachgiebige Element (6) dafür eingerichtet ist, für Nachgiebigkeit zu sorgen, wenn die medizinische Vorrichtung (5) in Bezug zu dem Anbringungssystem (7) bewegt wird,
**dadurch gekennzeichnet, dass** die medizinische Vorrichtung (5) eine medizinische Roboter-Vorrichtung und/oder eine Kathetersteuerungsvorrichtung umfasst.

2. Medizinisches System nach Anspruch 1, wobei das nachgiebige Element (6) ein Federelement umfasst.

3. Medizinisches System nach Anspruch 1, wobei das nachgiebige Element (6) dafür eingerichtet ist, Bewegungen der medizinischen Vorrichtung in Bezug auf das Anbringungssystem von mindestens 10 cm zu erlauben.

4. Medizinisches System nach Anspruch 1, wobei das Anbringungssystem (7) dafür eingerichtet ist, die medizinische Vorrichtung (5) derartig beweglich anzubringen, dass die medizinische Vorrichtung (5) an verschiedene Stellen und/oder in verschiedene Positionen bewegt werden kann.

5. Medizinisches System nach Anspruch 1, wobei die Referenzvorrichtung (9) ein Stützmittel zum Stützen des Lebewesens (23) ist, wobei das Befestigungselement (8) dafür eingerichtet ist, starr an dem Stützmittel angebracht zu werden.

6. Medizinisches System nach Anspruch 5, wobei das Stützmittel ein Patiententisch ist.

7. Medizinisches System nach Anspruch 1, wobei die medizinische Vorrichtung (5) für eine erste Sitzung durch das Befestigungselement (8) starr an der Referenzvorrichtung (9) angebracht werden kann, um für eine steife Verbindung zwischen der medizinischen Vorrichtung (5) und der Referenzvorrichtung (9) zu sorgen, wobei, nachdem die medizinische Vorrichtung von der Referenzvorrichtung gelöst wurde, die medizinische Vorrichtung (5) für eine zweite Sitzung durch das Befestigungselement (8) wieder starr an der Referenzvorrichtung (9) angebracht werden kann, um für eine steife Verbindung zwischen der medizinischen Vorrichtung (5) und der Referenzvorrichtung (9) zu sorgen, wobei das Befestigungselement (8) derartig eingerichtet ist, dass die räumliche Beziehung zwischen der medizinischen Vorrichtung (5) und der Referenzvorrichtung (9) in der ersten und der zweiten Sitzung die gleiche ist.

8. Medizinisches System nach Anspruch 1, wobei das medizinische System (1) weiterhin eine Bildgebungsvorrichtung (12) zum Erzeugen eines Bilds des Lebewesens (23) während der medizinischen Prozedur umfasst.

## Revendications

1. Système médical pour appliquer une procédure médicale à un être vivant, dans lequel le système médical (1) comprend :
- un dispositif médical (5) pour réaliser la procédure médicale, dans lequel le dispositif médical est adapté pour réaliser un déplacement par rapport à un dispositif de référence (9) pour appliquer la procédure médicale,
- un système d'attache (7) pour attacher le dispositif médical (5) à une paroi et/ou à un plafond (3) d'une pièce, dans lequel le système d'attache (7) est un système de suspension pour suspendre le dispositif médical (5) à la paroi et/ou au plafond (3) de la pièce (2),
- un élément de fixation (8) pour attacher solidement le dispositif médical (5) au dispositif de référence (9), et
- un élément de compliance passive (6) entre le système d'attache (7) et le dispositif médical (5), dans lequel l'élément de compliance (6) est adapté pour fournir une compliance lorsque le dispositif médical (5) est déplacé par rapport au système d'attache (7),
**caractérisé en ce que** le dispositif médical (5) inclut un dispositif robotique médical et/ou un dispositif de commande de cathéter.

2. Système médical selon la revendication 1, dans lequel l'élément de compliance (6) comprend un élément ressort.

3. Système médical selon la revendication 1, dans lequel l'élément de compliance (6) est adapté pour permettre des déplacements du dispositif médical par rapport au système d'attache d'au moins 10 cm.

4. Système médical selon la revendication 1, dans lequel le système d'attache (7) est adapté pour attacher de façon mobile le dispositif médical (5) de sorte que le dispositif médical (5) est mobile vers différents emplacements et/ou dans différentes positions.

5. Système médical selon la revendication 1, dans lequel le dispositif de référence (9) est un moyen de support pour supporter l'être vivant (23), dans lequel l'élément de fixation (8) est adapté pour être fixé solidement au moyen de support.

6. Système médical selon la revendication 5, dans lequel le moyen de support est une table pour patient.

7. Système médical selon la revendication 1, dans lequel, pendant une première session, le dispositif médical (5) peut être attaché solidement au dispositif de référence (9) par l'élément de fixation (8) pour assurer une liaison ferme entre le dispositif médical (5) et le dispositif de référence (9), dans lequel, après que le dispositif médical a été détaché du dispositif de référence, pendant une seconde session, le dispositif médical (5) peut à nouveau être attaché solidement au dispositif de référence (9) par l'élément de fixation (8) pour assurer une liaison ferme entre le dispositif médical (5) et le dispositif de référence (9), dans lequel l'élément de fixation (8) est adapté de sorte que la relation spatiale entre le dispositif médical (5) et le dispositif de référence (9) est la même dans les première et seconde sessions.

8. Système médical selon la revendication 1, dans lequel le système médical (1) comprend en outre un dispositif d'imagerie (12) pour générer une image de l'être vivant (23) durant la procédure médicale.
